# EUROPEAN PATENT SPECIFICATION

(11) **EP 4 326 899 B1**
(45) Date of publication and mention of the grant of the patent: **13.11.2024**
(21) Application number: 22724653.5
(22) Date of filing: 19.04.2022
(51) Int. Cl.: C12Q 1/6853

(54) **COMPOSITIONS AND METHODS FOR CELL-FREE NUCLEIC ACID ISOLATION**
ZUSAMMENSETZUNGEN UND VERFAHREN ZUR ZELLFREIEN NUKLEINSÄUREISOLIERUNG
COMPOSITIONS ET MÉTHODES D'ISOLEMENT D'ACIDES NUCLÉIQUES SANS CELLULES

(30) Priority: 20.04.2021 US 202163177315 P
(43) Date of publication of application: 28.02.2024
(73) Proprietor: SIMSen Diagnostics AB, 431 83 Mölndal (SE)
(72) Inventor: GODFREY, Tony, Chestnut Hill, Massachusetts 02467 (US); STÅHLBERG, Anders, 42834 Kållered (SE); SJÖBACK, Robert, 43332 Partille (SE); KUBISTA, Mikael, 43136 Mölndal (SE); JOHANSSON, Gustav, 42151 Västra Frölunda (SE)
(74) Representative: Barker Brettell Sweden AB
(86) International application number: PCT/EP2022/060315
(87) International publication number: WO 2022/223561

(56) References cited:
- WO-A1-2015/164212
- WO-A1-2016/138080
- WO-A1-2019/140298
- WO-A1-2020/190509
- CN-A- 107 893 100
- PETER ANDROVIC ET AL: "Two-tailed RT-qPCR: a novel method for highly accurate miRNA quantification", NUCLEIC ACIDS RESEARCH, vol. 45, no. 15, 13 July 2017 (2017-07-13), GB, pages e144 - e144, XP055517760, ISSN: 0305-1048, DOI: 10.1093/nar/gkx588
- JESSE J. SALK ET AL: "Enhancing the accuracy of next-generation sequencing for detecting rare and subclonal mutations", NATURE REVIEWS GENETICS, vol. 19, no. 5, 26 March 2018 (2018-03-26), GB, pages 269 - 285, XP055681812, ISSN: 1471-0056, DOI: 10.1038/nrg.2017.117

## Description

### BACKGROUND

Circulating cell-free nucleic acids (cfNA), which include cell-free DNA (cfDNA) have been shown to be useful in the diagnosis and characterization of conditions as diverse as pregnancy, trauma, sepsis, myocardial infarction, stroke, metabolic disease, and cancer.

WO 2019/140298 discloses a composition comprising a primer that is (a) a loopable primer comprising a target-specific section, an adaptor section, and a stem-forming section, wherein the stem-forming section is hybridizable to a portion of the target-specific section to form a stem structure, or (b) a split primer comprising a first target-specific section, a second target-specific section, and an adaptor section positioned between the first target-specific section and the second target-specific section, or (c) a split-loopable primer comprising a first target-specific section, a second target-specific section, a stem-forming section positioned between the first target-specific section and the second target-specific section, and an adaptor section, or comprising a first adaptor section, a second adaptor section, a stem-forming section positioned between the first adaptor section and the second adaptor section, and a target-specific section. Also disclosed is a method for amplifying a target locus of interest from a template DNA, comprising at least two pre-amplification cycles using the loopable primer, the split primer and/or the split-loopable primer, wherein each amplification cycle comprises annealing the primer to the template DNA or pre-amplification product thereof and elongating the annealed primer. Further disclosed is a kit for amplifying a target locus of interest, comprising the loopable primer, the split primer, and/or the split-loopable primer.

Androvic et al., Two-tailed RT-qPCR: a novel method for highly accurate miRNA quantification, Nucleic Acids Research, 2017, 45(15): e144 discloses Two-tailed RT-qPCR, a two-step RT-qPCR with SYBR-green detection chemistry, for quantification of microRNA. The target-specific primer for reverse transcription is composed of two hemiprobes complementary to two different parts of the target miRNA, connected by a hairpin structure.

### SUMMARY

The small size of circulating cell-free nucleic acids such as cfDNA proposes challenges for traditional sequencing and amplification-using analysis protocols. Provided herein are methods and compositions for improved isolation and amplification of cell-free nucleic acids such as cfDNA and cfRNA.

The present invention is defined in the independent claims. Further embodiments of the invention are defined in the dependent claims.

In some aspects, the present disclosure provides for a method for processing a polynucleotide sequence, the method comprising: combining in a reaction mixture suitable for processing said polynucleotide sequence: (i) said polynucleotide sequence; and (ii) a stem-loop primer that comprises: a 5' arm sequence configured to hybridize to a complementary first end region of said target sequence; a stem-loop sequence containing a unique molecular identifier (UMI) and comprising a 5' stem sequence, a loop sequence containing the UMI and a 3' stem sequence; and a 3' arm sequence configured to hybridize to a second end region of said target sequence. According to the invention, said first end region of said polynucleotide sequence is a 5' region of said polynucleotide sequence and said second end region is a 3' region of said polynucleotide sequence. In some embodiments, said 5' arm sequence is substantially complementary to said first end region of said polynucleotide sequence. In some embodiments, said 3' arm is sequence is substantially complementary to said second end region of said polynucleotide sequence. In some embodiments, said 3' arm comprises at least 5-10 nucleotides perfectly complementary to said second end region of said polynucleotide sequence. In some embodiments, the 5' most 5-10 nucleotides of said 3' arm is perfectly complementary to said second end region of said polynucleotide sequence. In some embodiments, said target polynucleotide sequence comprises no fewer than 11 nucleotides between a 3' end of said 5' region of said polynucleotide sequence and a 5' end of 3' region of said polynucleotide sequence. In some embodiments, said 5' arm sequence is capable of hybridizing to a region of said polynucleotide sequence no fewer than 11 nucleotides from a 5' end of a region of said polynucleotide sequence to which said 3' arm sequence is capable of hybridizing. In some embodiments, said polynucleotide sequence comprises DNA. In some embodiments, a polynucleotide molecule comprising said target polynucleotide sequence is 100 or fewer, 75 or fewer, 50 or fewer, or 30 or fewer nucleotides in length. In some embodiments, a polynucleotide molecule comprising said target polynucleotide sequence is 30 or greater, 40 or greater, 50 or greater, or 60 or greater nucleotides in length. In some embodiments, a polynucleotide molecule comprising said target polynucleotide sequence is not an miRNA. In some embodiments, said 5' arm sequence comprises about 5-22, about 5-10, or about 20-22 nucleotides in length. In some embodiments, said 3' arm sequence comprises about 5-22, about 5-10, or about 20-22 nucleotides in length. In some embodiments, said 5' stem sequence or said 3' stem sequence are about 5-22, 5-10, or 20-22 nucleotides in length. In some embodiments, said loop sequence is about 6-50 nucleotides in length. In some embodiments, said target polynucleotide sequence comprises no fewer than 11, about 20, about 30, about 40, about 50, about 60, about 70, about 80, about 90, or about 100, or more nucleotides between a 3' end of said 5' region of said target DNA sequence and a 5' end of 3' region of said target sequence. In some embodiments, the method further comprises providing cell-free DNA comprising said target polynucleotide sequence. In some embodiments, providing cell-free DNA further comprises obtaining a serum or plasma sample from a subject. In some embodiments, the method further comprises incubating said reaction mixture under conditions suitable to produce extension products from said polynucleotide sequence. In some embodiments, the method further comprises sequencing said extension products. In some embodiments, said UMI comprises about 6-12 degenerate nucleotides.

In some aspects, the present disclosure provides for an oligonucleotide primer, comprising: a 5' arm sequence configured to hybridize to a first end region of a polynucleotide sequence; a stem-loop sequence containing a UMI and comprising a 5' stem sequence, a loop sequence containing the UMI and a 3' stem sequence; and a 3' arm sequence configured to hybridize to a second end region of said polynucleotide sequence. According to the invention, said first end region of said polynucleotide sequence is a 5' region of said polynucleotide sequence and said second end region is a 3' region of said polynucleotide sequence. In some embodiments, said 5' arm sequence is substantially complementary to said first end region of said polynucleotide sequence. In some embodiments, said 3' arm is sequence is substantially complementary to said second end region of said polynucleotide sequence. In some embodiments, said 3' arm comprises at least 5-10 nucleotides perfectly complementary to said second end region of said polynucleotide sequence. In some embodiments, the 5' most 5-10 nucleotides of said 3' arm is perfectly complementary to said second end region of said polynucleotide sequence. In some embodiments, said target polynucleotide sequence comprises no fewer than 11 nucleotides between said first end region of said polynucleotide sequence and a said second end of said polynucleotide sequence. In some embodiments, said 5' arm sequence is capable of hybridizing to a region of said polynucleotide sequence no fewer than 11 nucleotides from a 5' end of a region of said polynucleotide sequence to which said 3' arm sequence is capable of hybridizing. In some embodiments, said 5' arm sequence comprises about 5-22, about 5-10, or about 20-22 nucleotides in length. In some embodiments, said 3' arm sequence comprises about 5-22, about 5-10, or about 20-22 nucleotides in length. In some embodiments, said 5' stem sequence or said 3' stem sequence are about 5-22, about 5-10, or about 20-22 nucleotides in length. In some embodiments, said loop sequence is about 6-20 nucleotides in length. In some embodiments, said target polynucleotide sequence comprises no fewer than 11, about 20, about 30, about 40, about 50, about 60, about 70, about 80, about 90, or about 100, or more nucleotides between a 3' end of said 5' region of said target DNA sequence and a 5' end of 3' region of said target sequence.

In some aspects, the present disclosure provides for a library of oligonucleotide primers, comprising a plurality of primers according to the disclosure, wherein the plurality of primers comprises a plurality of distinct unique molecular identifiers (UMIs).

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

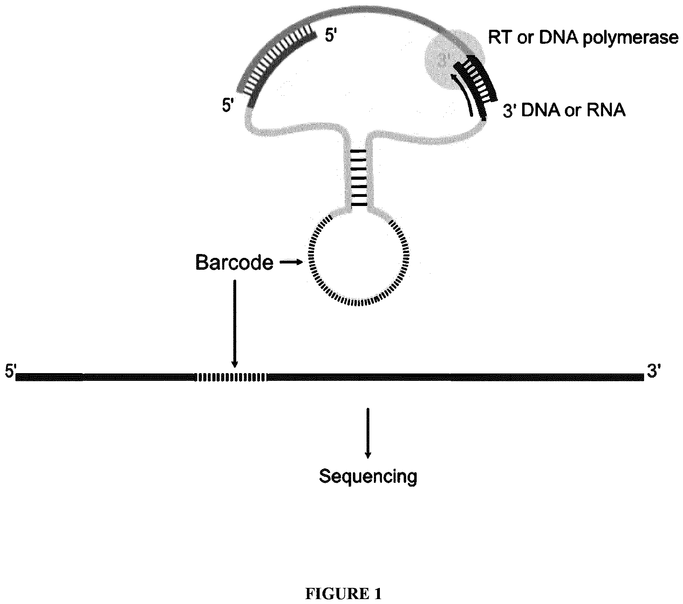

**FIGURE 1 (FIG.** 1) depicts a design of a two-tailed primer for cfNA according to some embodiments according to the disclosure. The top depiction shows the two-tailed primer in stem-loop form hybridized to a cell-free nucleic acid (e.g. cell-free DNA or cell-free RNA). The bottom depiction shows the organization of the regions of the stem-loop primer in linear fashion without secondary structure.

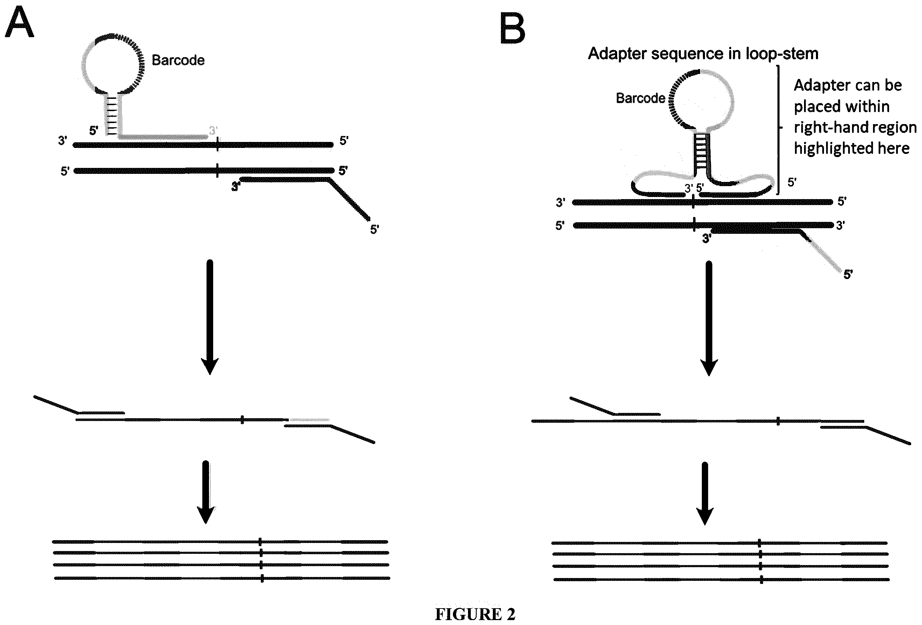

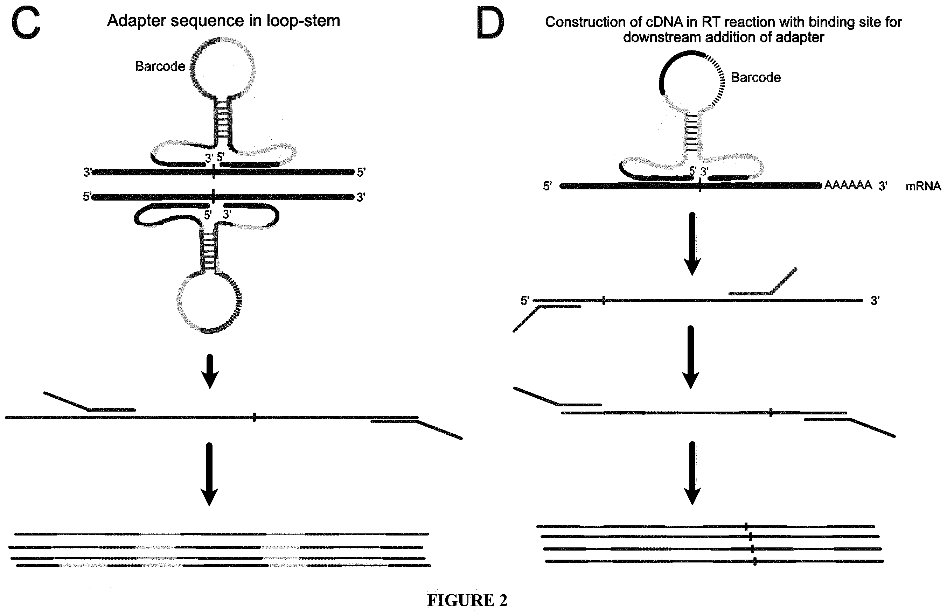

**FIGURE 2 (FIG. 2****)** illustrates possible designs for hemi-probe incorporating amplification primers or primer sets according to the disclosure. (A) Single-tailed 5' stem-loop primer in combination with a non-stem-loop 3' primer. (B) Stem-loop primer plus non-stem-loop primer: a two-tailed design with two hemiprobes (e.g. 10-15bp hemiprobes) are used to minimize the needed primer length for the 5' end primer , while a non-stem loop primer is as the 3' end primer; a sequencing adapter can further be incorporated as part of the stem loop of the 5' primer; (C) stem-loop primer plus stem loop primer; (D) Stem-loop primer for first single-stranded amplification step, followed by non-stem loop primer for a second single-stranded amplification step.

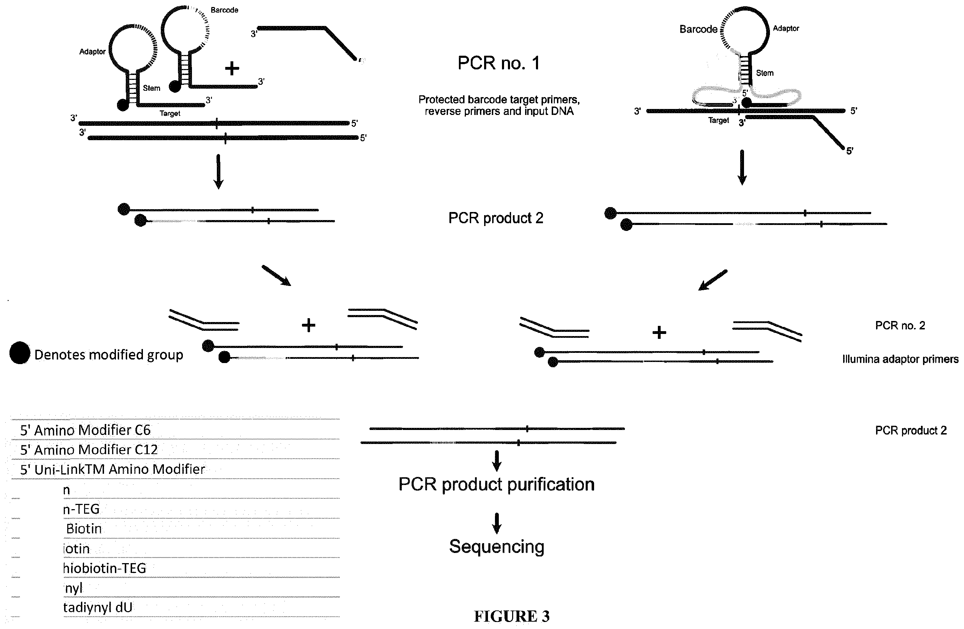

**FIGURE 3 (FIG. 3****)** shows an exemplary amplification protocol according to some embodiments of the invention using a hydrophobic group-modified (e.g. 5'-biotin-modified) stem-loop primer. For the single-probe stem-loop design (A), the hydrophobic group can be incorporated at the 5' most residue of the stem-loop. For the dual-hemiprobe design, the hydrophobic group can be incorporated at the 5' most region of the hemiprobe that binds the 3' most region of the half-sequence.

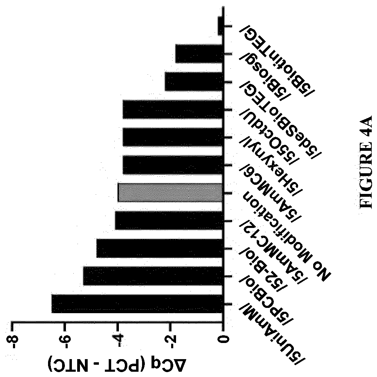

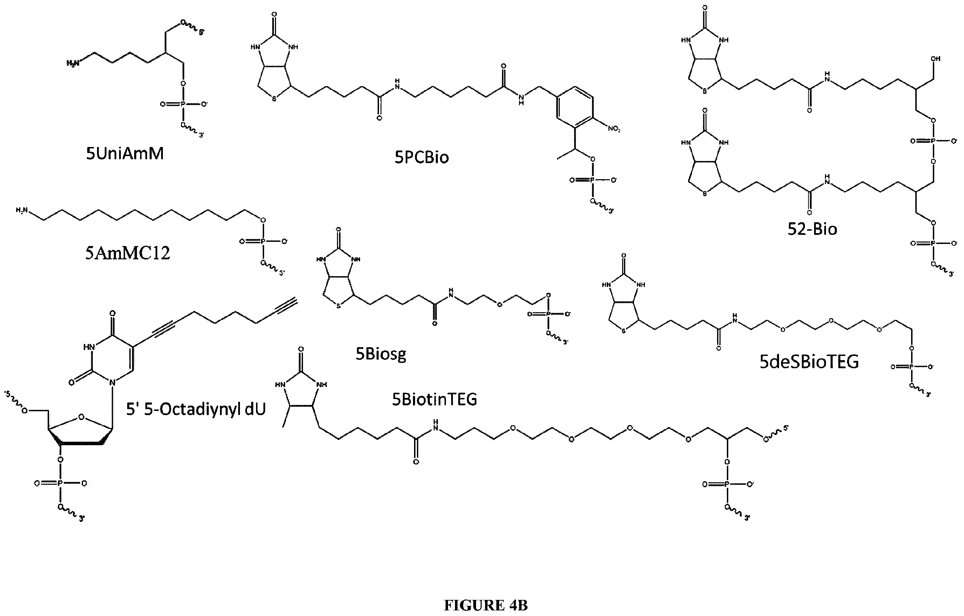

**FIGURES 4A** **and** **4B** **(****FIGs. 4A** **and** **4B****)** depict results of an amplification reaction as performed as depicted in the scheme of **FIGURE 3** using primers with or without various terminal hydrophobic groups to amplify samples containing ("positive") or not containing ("negative") a target DNA. **FIGURE 4A** shows the results of substituting the hydrophobic groups depicted in **FIGURE 4B** on the 5' end of the amplification primers; shown is RT-PCR difference in cycle time between "positive" and "negative" sample for different modifications.

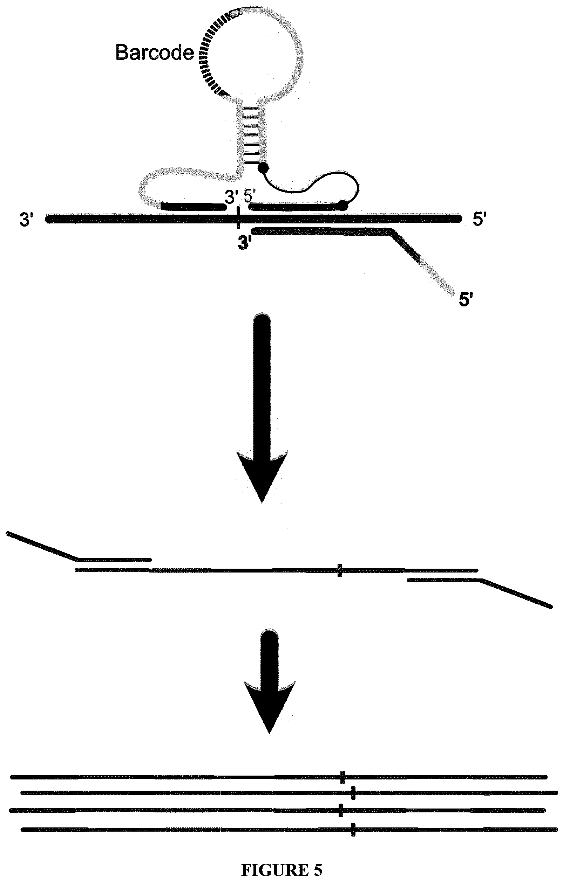

**FIGURE 5 (FIG. 5****)** depicts an example design of a two-hemiprobe primer containing an internal modification according to some embodiments of the disclosure

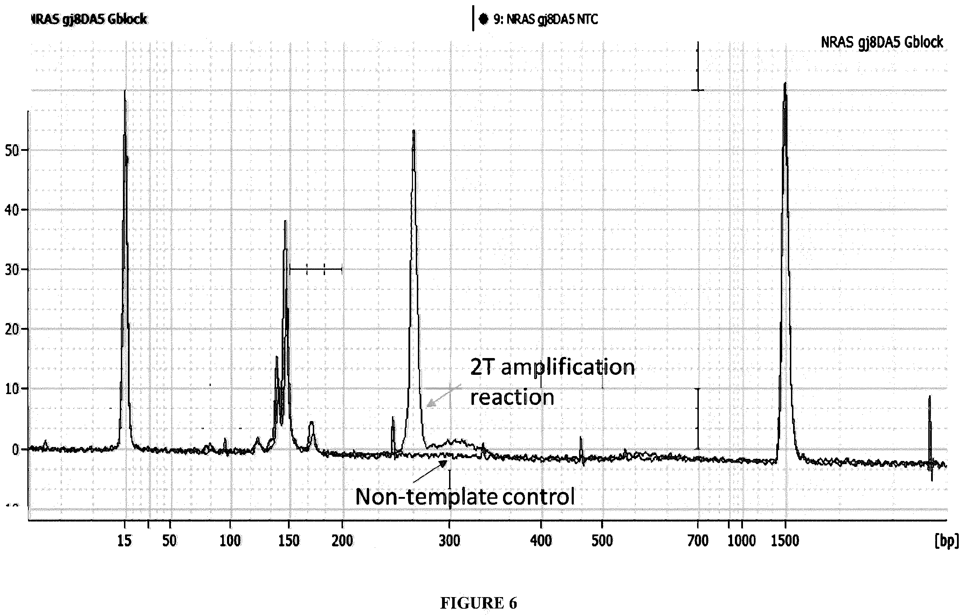

**FIGURE 6** shows the presence of an approximately 245 bp fragment in the reaction compared to a control that contained no synthetic template, demonstrating that the amplification reaction successfully created an amplification product containing the sequence flanked by the hemiprobe, the hemiprobe primer sequences, and the reverse primer sequences.

### DETAILED DESCRIPTION

The terminology used herein is for the purpose of describing particular cases only and is not intended to be limiting. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising."

The term "about" or "approximately" generally refers to an amount that is near the stated amount by about 10%, 5%, or 1%, including increments therein. For example, "about" or "approximately" can mean a range including the particular value and ranging from 10% below that particular value and spanning to 10% above that particular value.

The practice of some methods disclosed herein employ, unless otherwise indicated, techniques of immunology, biochemistry, chemistry, molecular biology, microbiology, cell biology, genomics, and recombinant DNA. See for example Sambrook and Green, Molecular Cloning: A Laboratory Manual, 4th Edition (2012); the series Current Protocols in Molecular Biology (F. M. Ausubel, et al. eds.); the series Methods In Enzymology (Academic Press, Inc.), PCR 2: A Practical Approach (M.J. MacPherson, B.D. Hames and G.R. Taylor eds. (1995)), Harlow and Lane, eds. (1988) Antibodies, A Laboratory Manual, and Culture of Animal Cells: A Manual of Basic Technique and Specialized Applications, 6th Edition (R.I. Freshney, ed. (2010)).

The term "nucleotide", as used herein, generally refers to a base-sugar-phosphate combination. A nucleotide may comprise a synthetic nucleotide. A nucleotide may comprise a synthetic nucleotide analog. Nucleotides may be monomeric units of a nucleic acid sequence (e.g., deoxyribonucleic acid (DNA) and ribonucleic acid (RNA)). The term nucleotide may include ribonucleoside triphosphates adenosine triphosphate (ATP), uridine triphosphate (UTP), cytosine triphosphate (CTP), guanosine triphosphate (GTP) and deoxyribonucleoside triphosphates such as dATP, dCTP, dITP, dUTP, dGTP, dTTP, or derivatives thereof. Such derivatives may include, for example, [αS]dATP, 7-deaza-dGTP and 7-deaza-dATP, and nucleotide derivatives that confer nuclease resistance on the nucleic acid molecule containing them. The term nucleotide as used herein may refer to dideoxyribonucleoside triphosphates (ddNTPs) and their derivatives. Illustrative examples of dideoxyribonucleoside triphosphates may include, but are not limited to, ddATP, ddCTP, ddGTP, ddITP, and ddTTP. In some cases, a nucleotide comprises particular chemical modifications to the nucleotide base. Such chemical modifications can be important e.g. for encoding of epigenetic information or post-transcriptional gene regulation. In some cases, such chemical modifications include methylation of the nucleotide base, and include 5-methylcytosine (5-mC), 5-hydroxymethylcytosine (5-hmC), 5-formylcytosine (5-fC) and 5-carboxylcytosine (5-caC). In some cases, such chemical modifications include adenosine deamination (e.g. conversion of adenosine to inosine) and cytosine deamination (e.g. conversion of cytosine to uracil).

The terms "polynucleotide," "oligonucleotide," and "nucleic acid" are used interchangeably to generally refer to a polymeric form of nucleotides of any length, either deoxyribonucleotides or ribonucleotides, or analogs thereof, either in single-, double-, or multi-stranded form. A polynucleotide may be exogenous or endogenous to a cell. A polynucleotide may exist in a cell-free environment. A polynucleotide may be a gene or fragment thereof. A polynucleotide may be DNA. A polynucleotide may be RNA. A polynucleotide may have any three-dimensional structure and may perform any function. A polynucleotide may comprise one or more analogs (e.g., altered backbone, sugar, or nucleobase). If present, modifications to the nucleotide structure may be imparted before or after assembly of the polymer. Some non-limiting examples of analogs include: 5-bromouracil, peptide nucleic acids, xeno nucleic acids, morpholinos, locked nucleic acids, glycol nucleic acids, threose nucleic acids, dideoxynucleotides, cordycepin, 7-deaza-GTP, fluorophores (e.g., rhodamine or fluorescein linked to the sugar), thiol containing nucleotides, biotin linked nucleotides, fluorescent base analogs, CpG islands, methyl-7-guanosine, methylated nucleotides, inosine, thiouridine, pseudourdine, dihydrouridine, queuosine, and wyosine. Non-limiting examples of polynucleotides include coding or non-coding regions of a gene or gene fragment, loci (locus) defined from linkage analysis, exons, introns, messenger RNA (mRNA), transfer RNA (tRNA), small nuclear RNA (snRNA), ribosomal RNA (rRNA), genomic DNA (gDNA), cell-free DNA (cfDNA), and circulating tumor DNA (ctDNA).

A "subject" from which a sample is derived can be prokaryote or a eukaryote. The eukaryote can be a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the subject or individual is a human.

In some cases, a sample is not from a subject, but is rather an environmental sample, comprising e.g. resident microorganisms or organic matter associated with higher organisms.

As used herein, the term "barcode" generally refers to a unique oligonucleotide sequence that allows a corresponding nucleic acid base and/or nucleic acid sequence, or a peptide or complex to which it is linked, to be identified. In some embodiments, the nucleic acid base and/or nucleic acid sequence is located at a specific position on a larger polynucleotide sequence, or a polynucleotide linked or connected to a peptide sequence. In some embodiments, a barcode is a "unique molecular identifier" (UMI). In some embodiments, barcodes can each have a length within a range of from 4 to 36 nucleotides, or from 6 to 30 nucleotides, or from 8 to 20 nucleotides. In some embodiments, the melting temperatures of barcodes within a set are within 10° C. of one another, within 5° C. of one another, or within 2° C. of one another. In some embodiments, barcodes are members of a minimally cross-hybridizing set (e.g., the nucleotide sequence of each member of such a set is sufficiently different from that of every other member of the set that no member can form a stable duplex with the complement of any other member under stringent hybridization conditions). In some embodiments, the nucleotide sequence of each member of a minimally cross-hybridizing set differs from those of every other member by at least two nucleotides. Example barcode technologies are described in Winzeler et al. (1999) Science 285:901; Brenner (2000) Genome Biol. 1:1 Kumar et al. (2001) Nature Rev. 2:302; Giaever et al. (2004) Proc. Natl. Acad. Sci. USA 101:793; Eason et al. (2004) Proc. Natl. Acad. Sci. USA 101 : 11046; and Brenner (2004) Genome Biol. 5:240.

As used herein, the term "cell-free DNA" or "cfDNA" generally refers to strands of deoxyribose nucleic acids (DNA) found free of cells, for example, as extracted or isolated from plasma/serum of circulating blood, extracted from lymph, cerebrospinal fluid (CSF), urine or other bodily fluids. In some cases "cfDNA" comprises "circulating tumor DNA" or "ctDNA", or cell-free DNA that is free of tumor origin.

In some embodiments, the nucleic acids used in methods described herein can be amplified. Amplification can be performed at any point during a multi reaction procedure, e.g., before or after pooling of sequencing libraries from independent reaction volumes and may be used to amplify any suitable target molecule described herein.

Amplification can be performed by any suitable method. The nucleic acids may be amplified by polymerase chain reaction (PCR), as described in, for example, U.S. Pat. Nos. 5,928,907 and 6,015,674. Other methods of nucleic acid amplification may include, for example, ligase chain reaction, oligonucleotide ligations assay, and hybridization assay, as described in greater detail in U.S. Pat. Nos. 5,928,907 and 6,015,674. Real-time optical detection systems are also known in the art, as also described in greater detail in, for example, U.S. Pat. Nos. 5,928,907 and 6,015,674. Other amplification methods that can be used herein include those described in U.S. Pat. Nos. 5,242,794; 5,494,810; 4,988,617; and 6,582,938.

Amplification may be achieved through any process by which the copy number of a target sequence is increased, e.g., PCR. Conditions favorable to the amplification of target sequences by PCR can be optimized at a variety of stages in the process, and can depend on characteristics of elements in the reaction, such as target type, target concentration, sequence length to be amplified, sequence of the target and/or one or more primers, primer length, primer concentration, polymerase used, reaction volume, ratio of one or more elements to one or more other elements, and others, some, or all of which can be altered. In general, PCR involves denaturation of the target to be amplified (if double stranded), hybridization of one or more primers to the target, and extension of the primers by a DNA polymerase, with the stages repeated (or "cycled") in order to amplify the target sequence. Stages in this process can be optimized for various outcomes, such as to enhance yield, decrease the formation of spurious products, and/or increase or decrease specificity of primer annealing. Methods of optimization are well known in the art and include adjustments to the type or amount of elements in the amplification reaction and/or to the conditions of a given stage in the process, such as temperature at a particular stage, duration of a particular stage, and/or number of cycles. In some embodiments, an amplification reaction comprises at least 5, 10, 15, 20, 25, 30, 35, 50, or more cycles. In some embodiments, an amplification reaction comprises no more than 5, 10, 15, 20, 25, 35, 50, or more cycles. Cycles can contain any number of stages, such as 1, 2, 3, 4, 5, 6, 7, 8, 9, 10 or more stages. Stages can comprise any temperature or gradient of temperatures, suitable for achieving the purpose of the given stage, including but not limited to, 3' end extension (e.g., adaptor fill-in), primer annealing, primer extension, and strand denaturation. Stages can be of any duration, including but not limited to about, less than about, or more than about 1, 5, 10, 15, 20, 25, 30, 35, 40, 45, 50, 55, 60, 70, 80, 90, 100, 120, 180, 240, 300, 360, 420, 480, 540, 600, or more seconds, including indefinitely until manually interrupted. Cycles of any number comprising different stages can be combined in any order. In some embodiments, different cycles comprising different stages are combined such that the total number of cycles in the combination is about, less that about, or more than about 5, 10, 15, 20, 25, 30, 35, 50, or more cycles.

### Samples

In some embodiments of the present disclosure, methods or systems described involve obtaining a sample from a subject or patient or involve the use of such a sample. Samples used in the methods of the provided disclosure can include, for example, a bodily fluid from a subject, including bile, blood and blood plasma, interstitial fluid, lymph, mucus (including snot and phlegm), pleural fluid, saliva, internal body fluids, including cerebrospinal fluid surrounding the brain and the spinal cord, synovial fluid surrounding bone joints, intracellular fluid inside cells, and vitreous humour in the eyeball. In one embodiment, the sample is a blood sample. The blood sample can be about 0.1, 0.2, 0.3, 0.4, 0.5, 0.6, 0.7, 0.8, 0.9, 1.0, 1.5, 2.0, 2.5, 3.0, 3.5, 4.0, 4.5, 5.0 mL, 10 ml, 20 ml, 30 ml, 40 ml, 50 ml, 100 ml, 200 ml, 300 ml, 400 ml, or more.

Human biofluids contain cells and also cell-free sources of molecules. Cell-free sources include extracellular vesicles and the molecules carried within (e.g. RNA, DNA, lipids, small metabolites, and proteins) and also cell-free DNA.

Samples can be collected and/or analyzed at a time when a disease of the subject is inactive. Samples can be collected and/or analyzed at a time when a disease of the subject is known to be active.

The sample can be obtained by a particular individual. The sample can be obtained by a health care provider, for example, a physician, physician assistant, nurse, veterinarian, dermatologist, rheumatologist, dentist, paramedic, or surgeon. The sample can be obtained by a research technician. More than one sample from a subject can be obtained.

As used herein "obtaining a sample" generally includes obtaining a sample directly or indirectly. In some embodiments, the sample is taken from the subject by the same party (e.g. a testing laboratory) that subsequently acquires biomarker data from the sample. In some embodiments, the sample is received (e.g. by a testing laboratory) from another entity that collected it from the subject (e.g. a physician, nurse, phlebotomist, or medical caregiver). In some embodiments, the sample is taken from the subject by a medical professional under direction of a separate entity (e.g. a testing laboratory) and subsequently provided to said entity (e.g. the testing laboratory). In some embodiments, the sample is taken by the subject or the subject 's caregiver at home and subsequently provided to the party that acquires biomarker data from the sample (e.g. a testing laboratory).

### Example embodiments

In some aspects, the present disclosure provides for a method for processing a polynucleotide sequence, the method comprising: combining in a reaction mixture suitable for processing said polynucleotide sequence: (i) said polynucleotide sequence; and (ii) a stem-loop primer. As used herein, the term "stem-loop" generally refers to a nucleic acid secondary structure in which a single strand of nucleic acid, e.g., DNA or RNA, has two self-complementary sequences, separated by some length of intervening sequence, such that the self-complementary sequences can hybridize to form a base paired "stem" connected by a "loop" made up of the non-base paired intervening sequence. The "stem" of a stem-loop will generally be of sufficient length to be stable in an amplification reaction (e.g. a PCR reaction) at the extension temperature used for the reaction. In some cases, stability of hybridization (or temperature of melting or Tₘ) is affected by the G/C and A/T content of the self-complementary sequences, and the length of the self-complementary sequences. In some cases, the stem of the stem loop is configured to hybridize only within particular temperature ranges (e.g. the Tₘ is altered such that the self-complementary sequences only hybridize to each other below a particular temperature). In some cases, the Tm of the interaction between the self-complementary (e.g. stem sequences) is between about 35°C and about 90°C, between about 40°C and about 75°C, between about 50°C and about 75°C , between about 45°C and about 65°C, or between about 50°C and about 55°C. In some cases, the Tm of the interaction between the self-complementary (e.g. stem sequences) is at least about 40°C, at least about 45°C, at least about 50°C, at least about 52°C, at least about 54°C, at least about 56°C, at least about 58°C, at least about 60°C, at least about 62°C, at least about 64°C, at least about 66°C, at least about 68°C, at least about 70°C, at least about 72°C, at least about 74°C, at least about 76°C, at least about 78°C, at least about 80°C, at least about 82°C, at least about 84°C, at least about 86°C, at least about 88°C, at least about 90°C, or more. In some cases, the Tm of the interaction between the self-complementary (e.g. stem sequences) is at most about 45°C, at most about 50°C, at most about 52°C, at most about 54°C, at most about 56°C, at most about 58°C, at most about 60°C, at most about 62°C, at most about 64°C, at most about 66°C, at most about 68°C, at most about 70°C, at most about 72°C, at most about 74°C, at most about 76°C, at most about 78°C, at most about 80°C, at most about 82°C, at most about 84°C, at most about 86°C, at most about 88°C, at most about 90°C, or more. In some cases, the self-complementary sequences are at least about 4, at least about 5, at least about 6, at least about 7, at least about 8, at least about 9, at least about 10, at least about 11, at least about 12, at least about 13, at least about 14, at least about 14, at least about 16, at least about 17, at least about 18, at least about 19, at least about 20, at least about 25, at least about 30, or more nucleotides in length. In some cases, the self-complementary sequences are no more than about 30, no more than about 25, no more than about 20, no more than about 19, no more than about 18, no more than about 17, no more than about 16, no more than about 15, no more than about 14, no more than about 13, no more than about 12, no more than about 10, no more than about 9, or no more than about 8 nucleotides in length. In some cases, the self-complementary sequences are at least about 4 to at least about 20 nucleotides in length. In some cases, the self-complementary sequences are at least about 6 to at least about 18 nucleotides in length. In some cases, the self-complementary sequences are at least about 8 to at least about 16 nucleotides in length. In some cases, the self-complementary sequences are at least about 10 to at least about 14 nucleotides in length. In some cases, the self-complementary sequences comprise a sequence having at least 90%, at least 95%, at least 96%, at least 97%, at least 98%, at least 99%, or a sequence that is substantially identical to at least one sequence from Table 1 or a complement or variant thereof.

In some cases, the stem-loop primer may comprise a nucleotide analogue, such as a modified nucleic acid, or other non-canonical nucleotide. Other non-limiting examples of nucleotide analogues include but are not limited to 2-Aminopurine, 2,6- Diaminopurine, 5-Bromo dU, deoxyUridine, Inverted dT, dideoxy nucleotides, 5-Methyl dC, deoxylnosine, locked nucleic acids (LNAs), 5-Nitroindole, 2'-O-Methyl RNA Bases, 2'-F RNA Bases, 5-bromouracil, peptide nucleic acids, xeno nucleic acids, morpholinos, glycol nucleic acids, threose nucleic acids, dideoxynucleotides, cordycepin, 7-deaza-GTP, fluorophores (e.g., rhodamine or fluorescein linked to the sugar), thiol containing nucleotides, biotin linked nucleotides, fluorescent base analogs, CpG islands, methyl-7-guanosine, methylated nucleotides, inosine, thiouridine, pseudourdine, dihydrouridine, queuosine, and wyosine.

In some embodiments, the polynucleotide sequence for processing is a sequence present in a nucleic acid mixture from a biological sample. Samples include any of the samples described herein. In some cases, the sample is a blood, peripheral blood, serum, or plasma sample. In some cases, the sample comprises DNA. In some cases, the sample comprises cell-free DNA (cfDNA). In some cases, the polynucleotide sequence comprises DNA. In some cases, the polynucleotide sequence comprises cfDNA or ctDNA. In some cases, plasma is purified by centrifugation to remove cellular matter before cfDNA isolation. In some cases, cfDNA is isolated from a blood, peripheral blood, serum, or plasma sample by silica membrane chromatography or polymer-mediated enrichment. The polynucleotide molecule comprising said target polynucleotide sequence can be 200 or fewer, 180 or fewer, 160 or fewer, 140 or fewer, 120 or fewer, 100 or fewer, 75 or fewer, 50 or fewer, 45 or fewer, 40 or fewer, 35 or fewer, or 30 or fewer nucleotides in length. The polynucleotide molecule comprising said target polynucleotide sequence can be 20 or greater, 25 or greater, 30 or greater, 35 or greater, 40 or greater, 45 or greater, 50 or greater, or 60 or greater nucleotides in length. In some cases, the polynucleotide molecule comprising the target polynucleotide comprises RNA. In some cases, the polynucleotide molecule comprising the target polynucleotide does not comprise RNA. In some cases, polynucleotide molecule comprising said target polynucleotide sequence is not an miRNA or snRNA. The polynucleotide molecule comprising said target polynucleotide sequence may comprise no fewer than about 9 nucleotides, no fewer than about 10 nucleotides, no fewer than about 11 nucleotides, no fewer than about 12 nucleotides, no fewer than about 13 nucleotides, no fewer than about 14 nucleotides, or no fewer than about 15 nucleotides between a 3' end (e.g. a first stem-loop arm hybridization site) of said 5' region of said polynucleotide sequence and a 5' end of 3' region (e.g. a second stem-loop arm hybridization site) of said polynucleotide sequence. In some cases, the biological sample is from any of the subjects described herein.

In some cases, the stem-loop primer comprises a 5' arm sequence configured to hybridize to a first end region of the target sequence. As used herein, the term "hybridize" generally refers to the physical interaction between complementary regions of two single-stranded nucleic acid molecules creating a double-stranded structure. In some cases, "hybridize" refers to promoting interactions between present oligonucleotides and their target polynucleotides under hybridization conditions that allow complementary regions of the two molecules to interact by hydrogen bonding and remain engaged. Modifiable variables of the hybridization conditions include, but are not limited to, duration (typically from seconds to some hours), temperature (generally from about 25°C to about 78°C), salt composition and concentration, chaotropic agent composition (e.g., formamide, or dimethyl sulfoxide (DMSO)) and concentration, and usage of substances that decrease non-specific binding (e.g., bovine serum albumin (BSA), or salmon sperm DNA (ssDNA)). In some cases, the 5' arm sequence is substantially complementary to said first end region of said polynucleotide sequence. The 5' arm can comprise at least about 5, at least about 6, at least about 7, at least about 8, at least about 9, at least about 10, at least about 11, at least about 12, at least about 13, at least about 14, at least about 15, at least about 16, at least about 17, at least about 18, at least about 19, at least about 20, at least about 21, or at least about 22 nucleotides perfectly complementary to said second end region of said polynucleotide sequence. The 5' arm can comprise no fewer than about 11 nucleotides, about 12 nucleotides, about 13 nucleotides, about 14 nucleotides, about 15 nucleotides, about 16 nucleotides, about 17 nucleotides, about 18 nucleotides, about 19 nucleotides, or about 20 nucleotides. In some cases, the 5' arm sequence comprises about 5-22, about 5-10, or about 20-22 nucleotides in length. The 5' arm may be configured (e.g. by length, complementarity, nucleotide composition, or addition of non-natural nucleotides) such that the sequence to which it is substantially complementary can fall within a particular temperature range, such as between about 35°C and about 90°C, between about 40°C and about 75°C, between 45°C and 65°C, or between 50°C and 55°C. In some cases, the 5' arm may be configured (e.g. by length, complementarity, nucleotide composition, or addition of non-natural nucleotides) to have a lower Tm with its complementary sequence than the Tm of the "stem" sequence (e.g. of the self-complementary sequences in the stem). In some cases, the Tm of the 5' arm with its complementary sequence (or the combined Tm of the 5' and 3' hemiprobes with their complementary sequence, if a two-hemiprobe primer design is used) is at least 1°C, 2°C, 3°C, 4°C, 5°C, 6°C, 7°C, 8°C, 9°C, 10°C, 12°C, 14°C, 16°C, 18°C, 20°C, 22°C, 24°C, 26°C, 28°C, 30°C, 32°C, 34°C, 36°C, 38°C, 40°C, or more degrees lower than the Tm of the stem sequence. In some cases, the Tm of the 5' arm with its complementary sequence (or the combined Tm of the 5' and 3' hemiprobes with their complementary sequence, if a two-hemiprobe primer design is used) is at least 10-20°C lower than the Tm of the stem sequence. The 5' most 5-10 nucleotides of said 5' arm may be perfectly complementary to said first end region of said polynucleotide sequence. In some cases, the primer comprises two hemiprobes,

In some cases, the stem-loop primer further comprises a loop sequence containing a barcode. In some cases, the barcode is a unique molecular identifier (UMI). The term "UMI", or "Unique Molecular Identifier", as used herein, generally refers to a tag, consisting of a sequence of degenerate bases, which is used to label original molecules in a sheared nucleic acid sample. As such, UMIs can be used to determine if two, similar sequence reads are each derived from a different, original fragment or if they are simply duplicates created during PCR amplification of the library, which were derived from the same original fragment. In some cases, UMIs can be used in combination with start stop sites, for consensus calling of rare sequence variants. In some cases, a UMI comprises about 6-12 degenerate nucleotides. In some cases, a UMI comprises at least about 6, at least about 7, at least about 8, at least about 9, at least about 10, at least about 11, at least about 12, at least about 13, at least about 14, at least about 15, at least about 16, at least about 17, at least about 18, at least about 19, or at least about 20 degenerate nucleotides. In some cases, a UMI comprises at most about 7, at most about 8, at most about 9, at most about 10, at most about 11, at most about 12, at most about 13, at most about 14, at most about 15, at most about 16, at most about 17, at most about 18, at most about 19, or at most about 20 degenerate nucleotides. In some cases, the UMI is flanked by a constant universal sequence to facilitate interrogation at later sequencing steps.

In some cases, the stem-loop primer further comprises a 3' arm sequence configured to hybridize to a second end region of said target sequence. In some cases, the 3' arm is sequence is substantially complementary to said second end region of said polynucleotide sequence. The 3' arm can comprise at least about 5, at least about 6, at least about 7, at least about 8, at least about 9, at least about 10, at least about 11, at least about 12, at least about 13, at least about 14, at least about 15, at least about 16, at least about 17, at least about 18, at least about 19, at least about 20, at least about 21, or at least about 22 nucleotides perfectly complementary to said second end region of said polynucleotide sequence. The 3' arm can comprise no fewer than about 11 nucleotides, about 12 nucleotides, about 13 nucleotides, about 14 nucleotides, about 15 nucleotides, about 16 nucleotides, about 17 nucleotides, about 18 nucleotides, about 19 nucleotides, or about 20 nucleotides. In some cases, the 3' arm sequence comprises about 5-22, about 5-10, or about 20-22 nucleotides in length. The 3' arm may be configured (e.g. by length, complementarity, nucleotide composition, or addition of non-natural nucleotides) such that the sequence to which it is substantially complementary can fall within a particular temperature range, such as between about 35°C and about 90°C, between about 40°C and about 75°C, between 45°C and 65°C, or between 50°C and 55°C. In some cases, the 3' arm (or in the case of a primer with two hemiprobes, the combined 5 ' and 3 ' hemiprobe) may be configured (e.g. by length, complementarity, nucleotide composition, or addition of non-natural nucleotides) to have a lower Tm with its complementary sequence than the Tm of the "stem" sequence (e.g. of the self-complementary sequences in the stem). In some cases, the Tm of the 3' arm with its complementary sequence is at least 1°C, 2°C, 3°C, 4°C, 5°C, 6°C, 7°C, 8°C, 9°C, 10°C, 12°C, 14°C, 16°C, 18°C, 20°C, 22°C, 24°C, 26°C, 28°C, 30°C, 32°C, 34°C, 36°C, 38°C, 40°C, or more degrees lower than the Tm of the stem sequence. In some cases, the 3' arm may be configured to have a Tm with its complementary sequence substantially equal to the Tm of the 5' arm with its complementary sequence, or a Tm within 1°C, 2°C, 4°C, 6°C, 8°C, or 10°C of the Tm of the 5' arm with its complementary sequence. The 5' most 5-10 nucleotides of the 3' arm may perfectly be complementary to said second end region of said polynucleotide sequence. In some cases, said 3' arm sequence is capable of hybridizing to a region of said polynucleotide sequence no fewer than about 9 nucleotides, no fewer than about 10 nucleotides, no fewer than about 11 nucleotides, no fewer than about 12 nucleotides, no fewer than about 13 nucleotides, no fewer than about 14 nucleotides, or no fewer than about 15 nucleotides from a 3' end of a region of said polynucleotide sequence to which said 3' arm sequence is capable of hybridizing. In some cases, the first end region of said polynucleotide sequence is a 5' region of said polynucleotide sequence and said second end region is a 3' region of said polynucleotide sequence.

In some cases, the method further comprises incubating said reaction mixture under conditions suitable to produce extension products from said polynucleotide sequence. Conditions suitable to produce extension products include but are not limited to any conditions suitable to extend a primer (e.g. a stem-loop primer) described herein in a template-directed manner. Such conditions include any amplification method described herein, RT-PCR, RT-qPCR, qPCR, PCR, LAMP, SDA, recombinase polymerase amplification, sanger sequencing, or RNA transcription. In some cases, incubating the reaction mixture under conditions to produce extension products from said polynucleotide sequence (e.g. in the presence of a stem-loop primer described herein) causes production of extension products from the polynucleotide sequence with particular characteristics). The extension products produced may have a particular purity, such as at least about 1%, at least about 2%, at least about 5%, at least about 8%, at least about 10%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 82%, at least about 84%, at least about 86%, at least about 88%, at least about 90%, at least about 92%, at least about 94%, at least about 96%, at least about 98%, at least about 99%, at least about 99.2%, at least about 99.4%, at least about 99.6%, or at least about 99.8% products containing said polynucleotide sequence.

In some cases, the method further comprises performing a sequencing assay on the extension products produced in the reaction mixture. The sequencing assay may comprise (i) exome sequencing, (ii) sequencing a panel of genes, (iii) whole genome sequencing, (iv) sequencing by synthesis using reversible terminator chemistry, (v) pyrosequencing, (vi) nanopore sequencing, (vii) real-time single molecule sequencing, (viii) sanger sequencing, or any combination thereof. Sequencing can be performed by various systems currently available, such as, without limitation, a sequencing system by Illumina^{®}, Pacific Biosciences (PacBio^{®}), Oxford Nanopore^{®}, or Life Technologies (Ion Torrent^{®}). Alternatively or in addition, sequencing may be performed using nucleic acid amplification, polymerase chain reaction (PCR) (e.g., digital PCR, quantitative PCR, or real-time PCR), or isothermal amplification.

In some aspects, the present disclosure provides for a stem-loop primer as described herein, a reaction mixture suitable for DNA amplification, or a reaction mixture suitable for production of extension products comprising a stem-loop primer as described herein.

In some aspects, the present disclosure provides for a library of oligonucleotide primers, comprising a plurality of stem-loop primers as described herein, wherein the plurality of stem-loop primers comprises a plurality of distinct unique molecular identifiers (UMIs). The library can comprise at least about 1, 2, 25, 50, 100, 1000, 10×10⁵, 10×10⁶, 10×10⁷, 10×10⁸, or 10×10⁹ members, or more.

In some aspects, the present disclosure provides for a method for processing a polynucleotide sequence, the method comprising: combining in a reaction mixture suitable for extension of polynucleotide primers: (i) said polynucleotide sequence; and (ii) a primer that comprises: a terminal hydrophobic group. In some cases, the primer further comprises a unique molecular identifier (UMI) as described herein. In some cases, the primer further comprises a 3' arm sequence capable of hybridizing to a first end region of said polynucleotide sequence (e.g. a 3' arm sequence as described herein). In some cases, the primer further comprises a 5' arm sequence capable of hybridizing to a first end region of said polynucleotide sequence (e.g. a 5' arm sequence as described herein). In some cases, the primer is a stem-loop primer as described herein. In some cases, the stem-loop primer comprises a UMI or a barcode as described herein.

The hydrophobic group can comprise any group having hydrophobicity greater than the natural 5'-OH of a DNA polynucleotide. Such groups include, but are not limited to, the 5' Uni-Link^{™} Amino Modifier (5UniAmM), 5' PC Biotin (5PCBio), 5' Dual Biotin (52-Bio), 5' Amino Modifier C12 (5AmMC12), 5' Amino Modifier C6 (5AmMC6), 5' Hexynyl (5Hexynyl), 5' 5-Octadiynyl dU (550ctdU), 5' Desthiobiotin-TEG (5deSBioTEG), 5' Biotin (5biosg), or 5' Biotin-TEG (5BiotinTEG), or any of the modifying groups depicted in . In some embodiments, the hydrophobic group is 5' Desthiobiotin-TEG (5deSBioTEG), 5' Biotin (5biosg), or 5' Biotin-TEG (5BiotinTEG). The hydrophobic group can be 5' Desthiobiotin-TEG (5deSBioTEG). The hydrophobic group can be 5' Biotin (5biosg). The hydrophobic group can be 5' Biotin-TEG (5BiotinTEG) In some cases, the method further comprises incubating said reaction mixture under conditions suitable to produce extension products from said polynucleotide sequence, wherein (b) is performed in absence of purification of said amplification products. Conditions suitable to produce extension products include but are not limited to any conditions suitable to extend a primer (e.g. a stem-loop primer) described herein in a template-directed manner. Such conditions include any amplification method described herein, RT-PCR, RT-qPCR, qPCR, PCR, LAMP, SDA, recombinase polymerase amplification, sanger sequencing, or RNA transcription. In some cases, incubating the reaction mixture under conditions to produce extension products from said polynucleotide sequence (e.g. in the presence of a stem-loop primer described herein) causes production of extension products from the polynucleotide sequence with particular characteristics). The extension products produced may have a particular purity, such as at least about as at least about 1%, at least about 2%, at least about 5%, at least about 8%, at least about 10%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 82%, at least about 84%, at least about 86%, at least about 88%, at least about 90%, at least about 92%, at least about 94%, at least about 96%, at least about 98%, at least about 99%, at least about 99.2%, at least about 99.4%, at least about 99.6%, or at least about 99.8% products containing said polynucleotide sequence in the absence of purification. Alternatively or additionally, the extension products produced may comprise fewer than about 80%, fewer than about 75%, fewer than about 70%, fewer than about 65%, fewer than about 60%, fewer than about 55%, fewer than about 50%, fewer than about 45%, fewer than about 40%, fewer than about35%, fewer than about 30%, fewer than about 28%, fewer than about 26%, fewer than about 24%, fewer than about 22%, fewer than about 20%, fewer than about 18%, fewer than about 16%, fewer than about 14%, fewer than about 12%, fewer than about 10%, fewer than about 9%, fewer than about 8%, fewer than about 7%, fewer than about 6%, fewer than about 5%, fewer than about 4%, fewer than about 3%, fewer than about 2%, fewer than about 1%, fewer than about 0.8%, fewer than about 0.6%, fewer than about 0.5%, fewer than about 0.4%, fewer than about 0.2%, fewer than about 0.1%, or less products that do not contain said polynucleotide target sequence in the absence of purification.

The reaction mixture can comprise a second primer comprising a 5' arm sequence configured to hybridize to a second end region of said polynucleotide sequence (e.g. a 5' arm sequence as described herein). In some cases, the second primer comprises a terminal hydrophobic group. The hydrophobic group can comprise any group having hydrophobicity greater than the natural 5'-OH of a DNA polynucleotide. Such groups include, but are not limited to the 5' Uni-Link^{™} Amino Modifier (5UniAmM), 5' PC Biotin (5PCBio), 5' Dual Biotin (52-Bio), 5' Amino Modifier C12 (5AmMC12), 5' Amino Modifier C6 (5AmMC6), 5' Hexynyl (5Hexynyl), 5' 5-Octadiynyl dU (55OctdU), 5' Desthiobiotin-TEG (5deSBioTEG), 5' Biotin (5biosg), or 5' Biotin-TEG (5BiotinTEG).

In some embodiments, the polynucleotide sequence for processing is a sequence present in a nucleic acid mixture from a sample from a subject. Samples include any of the samples described herein. In some cases, the sample is a blood, peripheral blood, serum, or plasma sample. In some cases, the sample comprises DNA. In some cases, the sample comprises cell-free DNA (cfDNA). In some cases, the polynucleotide sequence comprises DNA. In some cases, the polynucleotide sequence comprises cfDNA or ctDNA. In some cases, plasma is purified by centrifugation to remove cellular matter before cfDNA isolation. In some cases, cfDNA is isolated from a blood, peripheral blood, serum, or plasma sample by silica membrane chromatography or polymer-mediated enrichment. The polynucleotide molecule comprising said target polynucleotide sequence can be 200 or fewer, 180 or fewer, 160 or fewer, 140 or fewer, 120 or fewer, 100 or fewer, 75 or fewer, 50 or fewer, 45 or fewer, 40 or fewer, 35 or fewer, or 30 or fewer nucleotides in length. The polynucleotide molecule comprising said target polynucleotide sequence can be 20 or greater, 25 or greater, 30 or greater, 35 or greater, 40 or greater, 45 or greater, 50 or greater, or 60 or greater nucleotides in length. In some cases, the polynucleotide molecule comprising the target polynucleotide comprises RNA. In some cases, the polynucleotide molecule comprising the target polynucleotide does not comprise RNA. In some cases, polynucleotide molecule comprising said target polynucleotide sequence is not an miRNA or snRNA. The polynucleotide molecule comprising said target polynucleotide sequence may comprise no fewer than about 9 nucleotides, no fewer than about 10 nucleotides, no fewer than about 11 nucleotides, no fewer than about 12 nucleotides, no fewer than about 13 nucleotides, no fewer than about 14 nucleotides, or no fewer than about 15 nucleotides between a 3' end (e.g. a first arm hybridization site) of said 5' region of said polynucleotide sequence and a 5' end of 3' region (e.g. a second arm hybridization site) of said polynucleotide sequence.

**Table 1: Sequences of Nucleotides Described Herein**

| **SEQ ID NO:** | **NAME** | **TYPE** | **SOURCE** | **SEQUENCE** |
|---|---|---|---|---|
| 1 | P53 forward primer | Nucleotide | Synthetic sequence | GGACACTCTT TCCCTACACG ACGCTCTTCC GATCTNNNNN NNNNNNNATG GGAAAGAGTG TCC GTGGCAAGTG GCTCCTGA |
| 2 | P53 reverse primer | Nucleotide | Synthetic sequence | GTGACTGGAG TTCAGACGTG TGCTCTTCCG ATCTCATGGG CGGCATGAAC |
| | | | | |
| 3 | GJ8 NRAS forward primer | Nucleotide | Synthetic sequence | TACAGTGCCA TGAGAGACCA TTATCCTGGA CACTCTTTCC CTACACGACG CTCTTCCGAT CTNNNNNNNN NNNNATGGGA AAGAGTGTCC TCTACGCTGG ACAAG |
| 4 | NRAS reverse primer | Nucleotide | Synthetic sequence | GTGACTGGAG TTCAGACGTG TGCTCTTCCG ATCTACACAA AGATCATCCT TTCAGAGA |
| 5 | NRAS synthetic sequence template | Nucleotide | Synthetic sequence | CAGCTGGACA AGTAATATAC AGTGCCATGA GAGACCAATA CATGAGGACT AATATAATAA TTATTTTCTCT GAAAGGATGA TCTTTGTGTTC TAGT |
| 6 | Illumina universal forward primer | Nucleotide | Synthetic sequence | AATGATACGG CGACCACCGA GATCTACACT CTTTCCCTAC ACGACGCTCT TCCGATCT |
| 7 | Illumina index reverse primer | Nucleotide | Synthetic sequence | CAAGCAGAAG ACGGCATACG AGATCGTGAT GTGACTGGAG TTCAGACGTG TGCTCTTCCG ATCT |

### EXAMPLES

### Example 1. -Example Sequencing Reaction with Two-Tailed Primer to Process cell-free DNA

Primers are constructed as depicted in **FIGURE 2A****:** a 5' stem-loop primer bearing a 5 ' stem-loop containing a barcode and a universal sequence (green) and a 3' hybridization region to a upstream region of a cfDNA sequence (orange); and a 3' primer having a hybridization region to a downstream region of a cfDNA(red) and a universal sequence (green). These primers are incubated under amplification conditions with a suitable sample containing the cfDNA sequence. The resulting amplification products are analyzed by sequencing. Sequence filtering on the barcode sequence allows for identification and quantitation of the amplicons resulting from the specific cfDNA amplification reaction.

### Example 2.- Amplification Efficiency with Hydrophobic-Group Modified Stem-loop Primer

Forward primers targeted against the p53 gene with the sequence 5'-GGACACTCTTTCCCTACACGACGCTCTTCCGATCTNNNNNNNNNNNNATGGGAAAGAGT GTCCGTGGCAAGTGGCTCCTGA-3' (SEQ ID NO: 1, where N denotes degenerate residues) were modified to bear the 5' modifications shown in **FIGURE 4B** and were used with reverse primer 5'-GTGACTGGAGTTCAGACGTGTGCTCTTCCGATCTCATGGGCGGCATGAAC -3' (SEQ ID NO: 2, which also targets the p53 gene) to amplify human genomic DNA (positive) and nuclease free water (negative). The results of this experiment are presented in **FIGURE 4A****,** which depicts the change in cycle time between samples containing the target sequence and samples not containing the target sequence.

### Example 3.- Example Sequencing Reaction with Two-tailed Primer to Process NRAS Sequence Primer Design and Overview

To demonstrate amplification of DNA to produce a barcoded sequence as described herein using a 2-tailed forward primer, a primer was designed according to **panel B** in **FIGURE** 2 to amplify a sequence present in the NRAS gene.

This primer (designated GJ8) comprises of the sequence 5'-TACAGTGCCATGAGAGACCA-TTATCCT-GGACACTCTTTCCCTACACGACGCTCTTCCGATCT-NNNNNNNNNNNN-AT-GGGAAAGAGTGTCC-TCTA-CGCTGGACAAG-3' (SEQ ID NO: 3) where dashes are not part of the sequence but use here to visually distinguish the elements in the 5' to 3' direction; a 5' hemiprobe, a spacer, an adapter, a molecular barcode, a splitter, a sequence complementary to a section of the adapter capable of forming a hairpin, a spacer, and a 3' hemiprobe).

A corresponding reverse primer was also designed according to the scheme outlined in **panel B** of **FIGURE 2****,** and comprised the sequence 5'-GTGACTGGAGTTCAGACGTGTGCTCTTCCGATCTACACAAAGATCATCCTTTCAGAGA-3' (SEQ ID NO: 4).

These two primers were used to amplify a synthetic DNA construct with similarities to the NRAS gene, which comprised the sequence 5'-CAGCTGGACAAGTAATATACAGTGCCATGAGAGACCAATACATGAGGACTAATATAAT AATTATTTTCTCTGAAAGGATGATCTTTGTGTTCTAGT-3' (SEQ ID NO: 5), where the first and second bolded elements denote the 3' and 5' hemiprobe binding sites, respectively, and the third bolded sequence denotes the binding site for the reverse primer. Using the reaction procedure detailed below, 400,000 synthetic DNA molecules were amplified in a barcoding reaction and further processed to create a sequencing library in a second PCR reaction using Illumina index adapters.

### Reaction Procedure

The amplification was assembled in a 10 µL reaction, containing forward and reverse primers above, synthetic template, 1x Platinum Superfi buffer, 0.1 U platinum Superfi Polymerase (both from Thermo Fisher Scientific), 0.2 mM dNTP (Sigma-Aldrich), 40 nM of two-tailed and reverse primers (Ultramere, IDT), and 0.5 M L-carnitine inner salt (Sigma-Aldrich).

The amplification reaction was run on a T100 Thermal cycler (Bio-Rad) with the following thermal program; 30 sec at 98 °C, followed by 3 cycles of amplification (98°C for 10 sec, 60°C for 6 min, 72°C for 30 sec), 15 min at 65°C and 15 min at 95°C.

At the start of the 15 min incubation step at 65°C, 20 µl of 45 ng/µL Streptomyces griseus protease (Sigma-Aldrich) solution dissolved in 1x TE-buffer (pH 8.0, Thermo Fisher Scientific) was added to each well, to attenuate and degrade the polymerase.

10 µl of the resultant amplified product were then amplified with Illumina Universal forward primer and Illumina index reverse primer (desalted, Sigma-Aldrich, table 1) in a 40 µL reaction containing, 1x Q5 Hot Start High-Fidelity Master Mix (New England BioLabs) and 400 nM of each primer using the following thermal program on a T100 Thermal cycler; 98°C for 3 min, 30 cycles of amplification; 98°C for 10 sec, 80°C for 1 sec, 72°C for 30 sec, 76°C for 30 sec, all with ramping at 0.2°C/s. The Illumina universal forward primer comprised the sequence 5'-AATGATACGGCGACCACCGAGATCTACACTCTTTCCCTACACGACGCTCTTCCGATCT-3' (SEQ ID NO: 6), and the Illumina index reverse primer comprised the sequence 5'-

CAAGCAGAAGACGGCATACGAGATCGTGATGTGACTGGAGTTCAGACGTGTGCTCTTCC
GATCT-3′ (SEQ ID NO: 7).

Libraries were checked on a 2100 Bioanalyzer (Agilent) using the DNA 1000 kit (Agilent) and analyzed using the 2100 Expert software.

### Results

The result of the experiment are presented in **FIGURE 6** (see arrows), which depicts an automated gel electrophoresis trace of the amplification reaction compared to a control that contained no synthetic template, showing the presence of an approximately 245bp fragment in the reaction after amplification, demonstrating that the amplification reaction successfully created an amplification product containing the targeted sequence flanked by the Illumina universal forward primer sequences, a molecular barcode, a splitter, a sequence complementary to a section of the adapter, a spacer, and the 3' hemiprobe on the 5' side and the reverse primer sequence and the illumina reverse primer sequence on the 3' side.

## Claims

1. An oligonucleotide primer, comprising:
a 5' arm sequence configured to hybridize to a first end region of a polynucleotide sequence;
a stem-loop sequence comprising a 5' stem sequence, a loop sequence containing a UMI, and a 3' stem sequence; and
a 3' arm sequence configured to hybridize to a second end region of said polynucleotide sequence, wherein said first end region of said polynucleotide sequence is a 5' region of said polynucleotide sequence and said second end region of said polynucleotide sequence is a 3' region of said polynucleotide sequence.

2. The oligonucleotide primer of claim 1, wherein
said 5' arm sequence is configured to hybridize to said first end region of said polynucleotide sequence with a 5' end of said 5' arm sequence hybridized to a 3' end of said first end region of said polynucleotide and a 3' end of said 5' arm sequence hybridized to a 5' end of said first end region of said polynucleotide; and
said 3' arm sequence is configured to hybridize to said second end region of said polynucleotide sequence with a 5' end of said 3' arm sequence hybridized to a 3' end of said second end region of said polynucleotide and a 3' end of said 3' arm sequence hybridized to a 5' end of said second end region of said polynucleotide.

3. The oligonucleotide primer of claim 1 or 2, wherein
said 5' arm sequence is substantially complementary to said first end region of said polynucleotide sequence; and
said 3' arm is sequence is substantially complementary to said second end region of said polynucleotide sequence.

4. The oligonucleotide primer of any one of claims 1 to 3, wherein
the 5' arm sequence has a lower Tm with the first end region of the polynucleotide sequence than the 5' stem sequence with the 3' stem sequence; and
the 3' arm sequence has a lower Tm with the second end region of the polynucleotide sequence than the 5' stem sequence with the 3' stem sequence.

5. The oligonucleotide primer of any one of claims 1-4, wherein
said 5' arm sequence comprises 5-22, 5-10, or 20-22 nucleotides in length;
said 3' arm sequence comprises 5-22, 5-10, or 20-22 nucleotides in length;
said 5' stem sequence and said 3' stem sequence are 5-22, 5-10, or 20-22 nucleotides in length; and
said loop sequence is 6-20 nucleotides in length.

6. The oligonucleotide primer of any one of claims 1-5, wherein said oligonucleotide primer comprises a terminal hydrophobic group, preferably biotin or an analog or derivative thereof.

7. A method for processing a polynucleotide sequence, the method comprising:
combining in a reaction mixture suitable for processing said polynucleotide sequence:
(i) said polynucleotide sequence; and
(ii) an oligonucleotide primer of any one of claims 1-6.

8. The method of claim 7, wherein said first end region of said polynucleotide sequence is a 5' region of said polynucleotide sequence and said second end region is a 3' region of said polynucleotide sequence.

9. The method of claim 8, wherein said target polynucleotide sequence comprises no fewer than 11 nucleotides between a 3' end of said 5' region of said polynucleotide sequence and a 5' end of 3' region of said polynucleotide sequence.

10. The method of any one of claims 7-9, wherein said 5' arm sequence is capable of hybridizing to said first region of said polynucleotide sequence no fewer than 11 nucleotides from a 5' end of said second region of said polynucleotide sequence to which said 3' arm sequence is capable of hybridizing.

11. The method of any one of claims 7-10, wherein
a polynucleotide molecule comprising said target polynucleotide sequence is 100 or fewer nucleotides in length; and
said polynucleotide molecule comprising said target polynucleotide sequence is 30 or greater nucleotides in length.

12. The method of any one of claims 7-11, further comprising providing cell-free DNA comprising said target polynucleotide sequence.

13. The method of any one of claims 7-12, further comprising:
incubating said reaction mixture under conditions suitable to produce extension products from said polynucleotide sequence; and
optionally sequencing said extension products.

14. The method of claim 13, wherein
said oligonucleotide primer is of claim 6; and
incubating said reaction mixture under conditions suitable to produce extension products from said polynucleotide sequence is performed in absence of purification of said amplification products.

15. A library of oligonucleotide primers, comprising a plurality of oligonucleotide primers according to any one of claims 1-6, wherein the plurality of oligonucleotide primers comprises a plurality of distinct unique molecular identifiers (UMIs).

## Patentansprüche

1. Oligonukleotid-Primer, umfassend:
eine 5'-Armsequenz, die dazu konfiguriert ist, an einen ersten Endbereich einer Polynukleotidsequenz zu hybridisieren;
eine Stamm-Schleifen-Sequenz, die eine 5'-Stammsequenz, eine Schleifensequenz, die einen UMI enthält, und eine 3'-Stammsequenz umfasst; und
eine 3'-Armsequenz, die dazu konfiguriert ist, an einen zweiten Endbereich der Polynukleotidsequenz zu hybridisieren, wobei der erste Endbereich der Polynukleotidsequenz ein 5'-Bereich der Polynukleotidsequenz ist und der zweite Endbereich der Polynukleotidsequenz ein 3'-Bereich der Polynukleotidsequenz ist.

2. Oligonukleotid-Primer nach Anspruch 1, wobei
die 5'-Armsequenz dazu konfiguriert ist, an den ersten Endbereich der Polynukleotidsequenz mit einem 5'-Ende der 5'-Armsequenz, die an ein 3'-Ende des ersten Endbereichs des Polynukleotids hybridisiert ist, und einem 3'-Ende der 5'-Armsequenz, die an ein 5'-Ende des ersten Endbereichs des Polynukleotids hybridisiert ist, zu hybridisieren; und
die 3'-Armsequenz dazu konfiguriert ist, an den zweiten Endbereich der Polynukleotidsequenz mit einem 5'-Ende der 3'-Armsequenz, die an ein 3'-Ende des zweiten Endbereichs des Polynukleotids hybridisiert ist, und einem 3'-Ende der 3'-Armsequenz, die an ein 5'-Ende des zweiten Endbereichs des Polynukleotids hybridisiert ist, zu hybridisieren.

3. Oligonukleotid-Primer nach Anspruch 1 oder 2, wobei
die 5'-Armsequenz im Wesentlichen komplementär zu dem ersten Endbereich der Polynukleotidsequenz ist; und
die 3'-Armsequenz im Wesentlichen komplementär zu dem zweiten Endbereich der Polynukleotidsequenz ist.

4. Oligonukleotid-Primer nach einem der Ansprüche 1 bis 3, wobei die 5'-Armsequenz mit dem ersten Endbereich der Polynukleotidsequenz eine niedrigere Tm als die 5'-Stammsequenz mit der 3'-Stammsequenz aufweist; und
die 3'-Armsequenz mit dem zweiten Endbereich der Polynukleotidsequenz eine niedrigere Tm als die 5'-Stammsequenz mit der 3'-Stammsequenz aufweist.

5. Oligonukleotid-Primer nach einem der Ansprüche 1-4, wobei
die 5'-Armsequenz eine Länge von 5-22, 5-10 oder 20-22 Nukleotiden umfasst;
die 3'-Armsequenz eine Länge von 5-22, 5-10 oder 20-22 Nukleotiden umfasst;
die 5'-Stammsequenz und die 3'-Stammsequenz 5-22, 5-10 oder 20-22 Nukleotide lang sind; und
die Schleifensequenz 6-20 Nukleotide lang ist.

6. Oligonukleotid-Primer nach einem der Ansprüche 1-5, wobei der Oligonukleotid-Primer eine endständige hydrophobe Gruppe umfasst, vorzugsweise Biotin oder ein Analogon oder Derivat davon.

7. Verfahren zum Prozessieren einer Polynukleotidsequenz, wobei das Verfahren Folgendes umfasst:
in einem zum Prozessieren der Polynukleotidsequenz geeigneten Reaktionsgemisch Vereinigen:
(i) der Polynukleotidsequenz und
(ii) eines Oligonukleotid-Primers nach einem der Ansprüche 1-6.

8. Verfahren nach Anspruch 7, wobei der erste Endbereich der Polynukleotidsequenz ein 5'-Bereich der Polynukleotidsequenz ist und der zweite Endbereich ein 3'-Bereich der Polynukleotidsequenz ist.

9. Verfahren nach Anspruch 8, wobei die Zielpolynukleotidsequenz nicht weniger als 11 Nukleotide zwischen einem 3'-Ende des 5'-Bereichs der Polynukleotidsequenz und einem 5'-Ende des 3'-Bereichs der Polynukleotidsequenz umfasst.

10. Verfahren nach einem der Ansprüche 7-9, wobei die 5'-Armsequenz in der Lage ist, an den ersten Bereich der Polynukleotidsequenz nicht weniger als 11 Nukleotide von einem 5'-Ende des zweiten Bereichs der Polynukleotidsequenz, an den die 3'-Arm-Sequenz in der Lage ist zu hybridisieren, zu hybridisieren.

11. Verfahren nach einem der Ansprüche 7-10, wobei
ein Polynukleotidmolekül, das die Zielpolynukleotidsequenz umfasst, 100 oder weniger Nukleotide lang ist; und
das Polynukleotidmolekül, das die Zielpolynukleotidsequenz umfasst, 30 oder mehr Nukleotide lang ist.

12. Verfahren nach einem der Ansprüche 7-11, ferner umfassend Bereitstellen von zellfreier DNA, die die Zielpolynukleotidsequenz umfasst.

13. Verfahren nach einem der Ansprüche 7-12, ferner umfassend:
Inkubieren des Reaktionsgemisches unter Bedingungen, die geeignet sind, Extensionsprodukte aus der Polynukleotidsequenz herzustellen; und
optional Sequenzieren der Extensionsprodukte.

14. Verfahren nach Anspruch 13, wobei:
es sich um den Oligonukleotid-Primer nach Anspruch 6 handelt; und
Inkubieren des Reaktionsgemisches unter Bedingungen, die zum Herstellen von Extensionssprodukten aus der Polynukleotidsequenz geeignet sind, ohne Reinigung der Amplifikationsprodukte erfolgt.

15. Bibliothek von Oligonukleotid-Primern, umfassend eine Vielzahl von Oligonukleotid-Primern nach einem der Ansprüche 1-6, wobei die Vielzahl von Oligonukleotid-Primern eine Vielzahl von eindeutigen eindeutigen molekularen Identifikatoren (UMIs) umfasst.

## Revendications

1. Amorce oligonucléotidique, comprenant :
une séquence de bras 5' configurée pour s'hybrider à une première région d'extrémité d'une séquence polynucléotidique ;
une séquence de tige-boucle comprenant une séquence de tige 5', une séquence de boucle contenant un UMI et une séquence de tige 3' ; et
une séquence de bras 3' configurée pour s'hybrider à une seconde région d'extrémité de ladite séquence polynucléotidique, dans laquelle ladite première région d'extrémité de ladite séquence polynucléotidique est une région 5' de ladite séquence polynucléotidique et ladite seconde région d'extrémité de ladite séquence polynucléotidique est une région 3' de ladite séquence polynucléotidique.

2. Amorce oligonucléotidique selon la revendication 1, dans laquelle
ladite séquence de bras 5' est configurée pour s'hybrider à ladite première région d'extrémité de ladite séquence polynucléotidique avec une extrémité 5' de ladite séquence de bras 5' hybridée à une extrémité 3' de ladite première région d'extrémité dudit polynucléotide et une extrémité 3' de ladite séquence de bras 5' hybridée à une extrémité 5' de ladite première région d'extrémité dudit polynucléotide ; et
ladite séquence de bras 3' est configurée pour s'hybrider à ladite seconde région d'extrémité de ladite séquence polynucléotidique avec une extrémité 5' de ladite séquence de bras 3' hybridée à une extrémité 3' de ladite seconde région d'extrémité dudit polynucléotide et une extrémité 3' de ladite séquence de bras 3' hybridée à une extrémité 5' de ladite seconde région d'extrémité dudit polynucléotide.

3. Amorce oligonucléotidique selon la revendication 1 ou 2, dans laquelle
ladite séquence de bras 5' est sensiblement complémentaire de ladite première région d'extrémité de ladite séquence polynucléotidique ; et
ladite séquence de bras 3' est sensiblement complémentaire de ladite seconde région d'extrémité de ladite séquence polynucléotidique.

4. Amorce oligonucléotidique selon l'une quelconque des revendications 1 à 3, dans laquelle
la séquence de bras 5' a une Tm inférieure avec la première région d'extrémité de la séquence polynucléotidique que la séquence de tige 5' avec la séquence de tige 3' ; et
la séquence de bras 3' a une Tm inférieure avec la seconde région d'extrémité de la séquence polynucléotidique que la séquence de tige 5' avec la séquence de tige 3'.

5. Amorce oligonucléotidique selon l'une quelconque des revendications 1 à 4, dans laquelle
ladite séquence de bras 5' a une longueur de 5 à 22, 5 à 10 ou 20 à 22 nucléotides ;
ladite séquence de bras 3' a une longueur de 5 à 22, 5 à 10 ou 20 à 22 nucléotides ;
ladite séquence de tige 5' et ladite séquence de tige 3' ont une longueur de 5 à 22, 5 à 10 ou 20 à 22 nucléotides ; et
ladite séquence de boucle a une longueur de 6 à 20 nucléotides.

6. Amorce oligonucléotidique selon l'une quelconque des revendications 1 à 5, dans laquelle ladite amorce oligonucléotidique comprend un groupe hydrophobe terminal, de préférence la biotine ou un analogue ou un dérivé de celle-ci.

7. Procédé de traitement d'une séquence polynucléotidique, le procédé comprenant :
la combinaison dans un mélange réactionnel approprié pour traiter ladite séquence polynucléotidique :
(i) de ladite séquence polynucléotidique ; et
(ii) d'une amorce oligonucléotidique selon l'une quelconque des revendications 1 à 6.

8. Procédé selon la revendication 7, dans lequel ladite première région d'extrémité de ladite séquence polynucléotidique est une région 5' de ladite séquence polynucléotidique et ladite seconde région d'extrémité est une région 3' de ladite séquence polynucléotidique.

9. Procédé selon la revendication 8, dans lequel ladite séquence polynucléotidique cible comprend au moins 11 nucléotides entre une extrémité 3' de ladite région 5' de ladite séquence polynucléotidique et une extrémité 5' de la région 3' de ladite séquence polynucléotidique.

10. Procédé selon l'une quelconque des revendications 7 à 9, dans lequel ladite séquence de bras 5' est capable de s'hybrider à ladite première région de ladite séquence polynucléotidique ayant au moins 11 nucléotides d'une extrémité 5' de ladite seconde région de ladite séquence polynucléotidique à laquelle ladite séquence de bras 3' est capable de s'hybrider.

11. Procédé selon l'une quelconque des revendications 7 à 10, dans lequel
une molécule polynucléotidique comprenant ladite séquence polynucléotidique cible a une longueur de 100 nucléotides ou moins ; et
ladite molécule polynucléotidique comprenant ladite séquence polynucléotidique cible a une longueur de 30 nucléotides ou plus.

12. Procédé selon l'une quelconque des revendications 7 à 11, comprenant également la fourniture d'ADN acellulaire comprenant ladite séquence polynucléotidique cible.

13. Procédé selon l'une quelconque des revendications 7 à 12, comprenant également :
l'incubation dudit mélange réactionnel dans des conditions appropriées pour produire des produits d'extension à partir de ladite séquence polynucléotidique ; et
éventuellement le séquençage desdits produits d'extension.

14. Procédé selon la revendication 13, dans lequel
ladite amorce oligonucléotidique est selon la revendication 6 ; et
l'incubation dudit mélange réactionnel dans des conditions appropriées pour produire des produits d'extension à partir de ladite séquence polynucléotidique est réalisée en l'absence de purification desdits produits d'amplification.

15. Bibliothèque d'amorces oligonucléotidiques, comprenant une pluralité d'amorces oligonucléotidiques selon l'une quelconque des revendications 1 à 6, dans laquelle la pluralité d'amorces oligonucléotidiques comprend une pluralité d'identifiants moléculaires uniques distincts (UMI).
